Europäisches Patentamt

European Patent Office

Office européen des brevets

(19)

(11) Publication number: 0 403 292
A2

## EUROPEAN PATENT APPLICATION

(12)

(21) Application number: 90306526.6

(22) Date of filing: 14.06.90

(51) Int. Cl.5: **A23K 1/00, A23K 1/18**

(30) Priority: 14.06.89 GB 8913657

(43) Date of publication of application:
19.12.90 Bulletin 90/51

(84) Designated Contracting States:
AT BE CH DE DK ES FR GB GR IT LI LU NL SE

(71) Applicant: MICROBIAL DEVELOPMENTS
LIMITED
Spring Lane North
Malvern Link Worcestershire WR14 1AH(GB)

(72) Inventor: Day, Carol Avis
Hoppe Croft, 34 The Village
Clifton on Teme, Worcester, WR6 6DH(GB)
Inventor: Holton, Brian Wilson
Willow End Blackmore Park Road
Danemoor Malvern, Worcs. WR 14 3LF(GB)

(74) Representative: Ablewhite, Alan James et al
MARKS & CLERK 57/60 Lincoln's Inn Fields
London WC2A 3LS(GB)

(54) **Ruminant feedstuff additives.**

(57) The present invention relates to the problem of lactic acid bacteria in ruminants, the problem being overcome by the administration of the relevant bacteriophage(s).

EP 0 403 292 A2

## RUMINANT FEEDSTUFF ADDITIVES

The present invention relates to substances for the treatment and prophylaxis of low rumen pH-associated conditions, especially acidosis, in ruminants.

The great majority of rumen bacteria are obligate anaerobes, and can be divided into 7 main groups; cellulolytic, starch digesters, hemicellulose digesters, sugar fermenters, lactate utilisers, methanogenic and proteolytic bacteria. All groups either produce or utilise volatile fatty acids and normally provide a fairly consistent proportioning of acetate, propionate and butyrate in the rumen.

The volatile fatty acids produced in the rumen are almost completely absorbed by the animal and are metabolised after passage through the rumen wall. Removal of these acids at the rumen ensures that the rate of fermentation continues with little change in pH, which is usually within a range of pH 5.0-7.0, averaging pH 6.3 in a healthy animal. This is normally relatively constant, as volatile fatty acid absorption increases with acidity, thus preserving the status quo.

Forage, such as grass, maize, lucerne, arable, beans and peas, is harvested by farmers during the warmer months of the year and may be conserved by a technique known as ensiling. This technique involves containing the material and excluding air, to promote anaerobic fermentation of the sugars present in the fodder to generate lactic and other silage acids.

The structural and storage carbohydrates in forages and concentrates are broken down by the cellulolytic, hemicellulose- and starch-digesting rumen bacteria into easily-utilised sugars which, in turn, are converted to volatile fatty acids by the sugar fermenters. However, low-pH silages and high-energy concentrates can disrupt the stability of the rumen, in terms of microbial load, pH and volatile fatty acid composition, as low-pH silages already have a high lactic acid load and high-energy concentrates can lead to high lactic acid levels due to the action of the rumen bacteria.

The presence of excess lactic acid can be detected by the animal at the rumen wall and this can result in a direct depressive effect on feed intake. However, this sensory response is usually not sufficiently rapid to prevent the onset of acidosis, a condition which can ultimately cause the death of the animal.

Microbial action on lactic acid in the rumen contents means that it is eventually broken down to acetate, propionate and butyrate by appropriate biochemical pathways, but the overall level of lactic acid, once raised, remains high since the resultant lower pH favours the survival and rapid growth of acid-resistant bacteria, particularly lactic acid bacteria, such as Streptococcus bovis, which produce even more lactic acid, thereby perpetuating the low pH condition.

In addition, low pH and high levels of lactic acid also restrict the growth and enzymatic action of the cellulolytic bacteria, which prefer a higher pH of around 5.5-6.5. The retardation of their growth, in turn, also means that the fibre digestibility of the feedstuffs in the rumen is reduced.

As a result, ruminants fed on low-pH silage and/or high-energy, starch-based concentrates tend to show reduced intake of feed, increased incidence of acidosis, and reduced efficiency in terms of fibre and feedstuff digestion, due to rumen disturbance.

There is currently no satisfactory method for treating acidosis, as antibiotics will tend to reduce all the gut flora, which is potentially as disastrous as acidosis itself. Feeding cattle with sufficient antacid is an impractical solution, and so demand remains for a means for the treatment or prophylaxis of acidosis.

It has now been found that the use of specific bacteriophages to control certain rumen bacteria, particularly lactic acid bacteria, and especially Streptococcus bovis, reduces the production of lactic acid in the rumen and, so, can be used for the treatment or prophylaxis of low rumen-pH related conditions, especially acidosis, in ruminants, thereby maintaining or restoring rumen pH for efficient digestion of the cellulolytic and hemicellulolytic components of the diet.

Thus, in a first aspect of the present invention, there is provided a bacteriophage for use in ruminant therapy, particularly the use of at least one species of bacteriophage in food-stuffs or their ingredients for the treatment or prevention of low rumen pH-associated conditions in ruminants

In an alternative aspect of the present invention, there is provided a process for the preparation of a food-stuff comprising the addition of a preparation of at least one species of bacteriophage to the food-stuff or at least one of the ingredients therefor.

The present invention also provides preparations containing at least one variety of bacteriophage and a suitable carrier therefor for use according to either of the above described aspects of the invention.

The invention also provides a method for the cosmetic treatment of a ruminant, especially a cow, comprising administering at least one variety of bacteriophage specific for a lactic acid bacterium, especially S. equinus or E. durans, to said ruminant in a manner, such as orally, adapted to introduce the bacteriophage into the digestive tract, particularly the rumen, of the ruminant.

2

EP 0 403 292 A2

Also provided is a foodstuff, such as beef-grower nuts or water, or an ingredient thereof, for a ruminant, especially a cow, characterised in that the foodstuff contains at least one variety of bacteriophage specific for a lactic acid bacterium, especially S. equinus or E. durans.

In addition, there is provided a bacteriophage specific for a lactic acid bacterium, especially S. equinus or E. durans, for use in ruminant, especially cattle, therapy, particularly for the treatment of low rumen pH associated conditions, especially the treatment or prophylaxis of acidosis.

There is further provided the use of at least one variety of bacteriophage specific for a lactic acid bacterium, especially S. equinus or E. durans, in the manufacture of a medicament for the treatment or prophylaxis of low rumen pH associated conditions, especially acidosis, in ruminants, especially cattle.

The invention also includes a preparation of a bacteriophage as defined above, wherein the preparation is a foodstuff containing about $10^2$ to about $10^{12}$ pfu/gm (plaque forming units/gm) of bacteriophage, and a preparation of a bacteriophage for use as above, wherein the preparation is adapted for the administration of between about $10^9$ and about $10^{12}$ especially about $10^{10}$, pfu/animal/day of bacteriophage.

There is yet further provided a composition of the above preparations, wherein there are at least two preparations which contain a differing selection of bacteriophage.

Low-pH silages cause the lactic acid level to remain high, owing to a favourable pH environment for the growth of Streptococcus bovis and other lactic acid bacteria, leading to high levels of lactic acid production. Accordingly, use of specific bacteriophages in accordance with the present invention can retard the growth of Streptococcus bovis and other undesirable lactic acid bacteria, thus allowing other rumen micro-organisms to utilise the excess lactic acid, converting it to acetate, propionate and butyrate. The overall level of lactic acid therefore decreases and the rumen pH can return to normal.

Lactic acid bacteria in general, and Streptococcus bovis in particular, can take advantage of the high levels of available carbohydrate in high-energy and starch-based concentrates to produce high levels of lactic acid. In such an instance also, the use of specific bacteriophages can help to reduce overproduction of lactic acid, and thus stop the depression of pH to a level where normal rumen function is disturbed.

Other advantages accruing from the present invention through the reduction of the overall level of lactic acid in the rumen are;

a) cellulolytic micro-organisms are not inhibited and efficient fibre digestion is maintained;

b) feed intake is not depressed since lower lactic acid levels are sensed at the rumen wall and more efficient digestion of cellulose and hemicelluose (fibre) is maintained, thereby improving the rate of passage through the rumen, leading to a higher dry matter intake; and

c) acidosis is reduced or eliminated.

Other advantages of the present invention lie especially in the fact that separate medication is not necessary and in that preparations of ' phage are inherently tasteless and odourless.

A particular advantage in the use of phages according to the present invention is that, especially as only extremely small quantities of phage are required for efficacy, there is no adverse effect on flavour. Furthermore, as phages only infect highly specific organisms, no propagation of phage can take place in the absence of the host bacterium, so the food-stuff remains completely unaffected by their presence.

Thus, in a further aspect of the present invention there is provided the use or process as defined above wherein the bacteriophage(s) is selected according to host bacterium specificity.

Bacteriophages are highly host-specific. Extremely rapid multiplication within the host cell occurs, leading to the destruction of the cell (lysis) and the release of up to 20,000 new phages, each capable of further infection. Phages are essentially non-living outside the host cell and, therefore, can exhibit very considerable longevity, making them particularly useful in preparations according to the present invention.

It will be appreciated that as bacteriophages are highly specific in the organisms they can infect, any one variety of phage will only infect one species of bacterium and, frequently, only selected strains of that species. There is, thus, no danger of humans or animals being infected by the phage, which will only activate if the target organism is present, and will attack that organism alone, becoming dormant once more on completion of the task.

It will be appreciated that, while lysogenic phages can be used in accordance with the invention, the use of lytic phages is generally preferred, as infection results in the rapid destruction of the target bacterium.

Bacteria are known to be able to develop resistance to phage infection. The present invention, therefore, further provides a use or process as described above wherein the preparation comprises at least two strains of phage specific for one host. If the target micro-organism develops a resistance to one phage, or the phage becomes lysogenic, elimination of the unwanted organism still occurs.

Still more preferable is the 'rotation' of phages. For example, in the case where three phages are available, then three preparations of different phage pairs are available for use in successive treatments to

3

minimise the risk of resistance developing.

As used herein, ' rotation' means varying the bacteriophages used in the treatment or preparations according to the present invention such that the animals do not always receive the same combination of bacteriophages. Such variation need not be cyclical, or even regular, provided that different compositions are used occasionally to prevent resistance developing.

Preparations according to the present invention may contain phages specific for several different species of bacterium. However, it is preferred that at least one strain of phage infect, or be specific for, S. bovis.

When preparing a food-stuff for use in accordance with the invention, a bacteriophage concentration of $10^2$ to $10^{12}$ pfu (plaque forming units), especially $10^{10}$, per gram of forage is generally suitable.

Addition of phage (preferably $10^3$ - $10^7$ pfu/ml) to drinking water and/or feedingstuffs is generally effective to control lactic acid bacteria in the rumen, and is an effective way of leading to useful weight gain (and milk production in cattle).

As indicated above, the phage preparation may be introduced into a feed-stuff directly, for example by spraying, soaking in a preparation or mixing in a suitably impregnated powder, or may be administered in the drinking water, or may be administered by any suitable oral route, including pellets and oral spray, for example.

Preparations according to the present invention suitably contain at least two antigenic types of phage, optionally specific for more than one family of bacterium, as required. The preparations may be liquid or solid according to requirements. Liquid preparations may be simple suspensions of phage in water, but preferably further comprise another suitable carrier. Suitable carriers may, for example, be sugar-based, such as mannitol, but may comprise any suitable substance known in the art.

Liquid preparations may be prepared from any of the preparations generally available for similar use, a suitable quantity of phage being added. In the alternative, a more concentrated ' stock' solution may be prepared for dilution and use as necessary.

Similar considerations to the above apply, mutatis mutandis, to solid preparations and formulations for administration to livestock. Solid preparations are usually available in powdered or granular form, and so any ' stock' solution must comprise a carrier, such as ground maize, bran or limestone.

Formulations for administration to livestock will usually be formulated for inclusion with the feed addition to drinking water. It will generally be the case that solid formulations are suitable for addition to fodder, while liquid formulations are suitable for addition to drinking water.

Formulations for administration to livestock may also be given directly in any suitable form, such as bolus, capsule, tablet or solution, together with vitamin and/or mineral mixtures, feed compounds, or after adding to silage during its preparation or feedout, or suitable combinations thereof, as desired.

Suitable bacteriophages for use according to the present invention may be isolated from soils, silages, silage effluents and farm slurry, animal and plant wastes, alimentary tract contents, air or water, preferably using known bacteriophage enhancement techniques [c.f. Betz, J.V., & Anderson, K.E., (1983), J. Bact., 87, 408]. Bacteriophages so isolated may be characterised as to their host specificity by known techniques.

Suitable quantities of phage for use according to the present invention may be obtained, for example, by a batch technique, wherein a culture of host bacterium is grown nearly to capacity and then seeded with phage. After a suitable time has elapsed to allow maximal phage propagation, the culture is further lysed by chemical or physical techniques, if required, and the lysate spun down. The phage-bearing supernatant may then be further purified, for example by ultrafiltration, and then concentrated (spray-drying, vacuum-drying or freeze-drying, for instance), if necessary. Bulking agents, such as dextrose, may be used in the concentrating process to aid in recovery of the concentrated phage. The resulting preparation can be used directly or further combined with other ingredients to aid in packaging, end-use etc.

Large-scale commercial production of phages may involve initial anaerobic fermentation of the host species, preferably in optimal submerged culture conditions for a time adequate to achieve logarithmic growth of the culture. Specific phage preparations are then introduced to the culture and incubation continued until maximal lysis can be demonstrated. Downstream recovery of such a phage preparation from solution may be effected by initial low-speed centrifugation to remove any remaining bacterial cells and debris, and the phage purified and concentrated by ultracentrifugation and ultrafiltration techniques. The resulting concentrated phage preparation can be cryoprotected and freeze-dried by techniques well-known in the art, or preferably plated onto, or mixed with, a suitable carrier material and air- or vacuum-dried as appropriate. Typical activity levels of phages prepared according to the above methods range from $10^9$ - $10^{12}$ pfu/gram of concentrate according to the particular phage morphology.

In order to determine phage activity in the final product, as well as during preparation, total specific phage counts may be undertaken using, for example, the double agar layer plating technique for plaque production, employing the host microbial species in each case. The method described by Adams, M. H., in "Bacteriophages" (Interscience Publishers (1959)) is suitable for this purpose.

The following Examples are for purposes of illustration only, and are not intended to limit the scope of the present invention in any way.

EXAMPLE 1

Formulations

a) Powdered limestone (Trucarb 52 [trade mark]) was mixed with spray-dried bacteriophages specific for S. bovis and for S. durans to a final mix of $1.2 \times 10^9$ pfu/gm. The product was then packaged in sachets of 120 gm.

b) Ultrafiltered bacteriophage (specificities as above) solution was blended with dextrose as a bulking agent, and spray-dried. The product was then packaged.

c) The spray-dried product of b) is used to prepare beef-grower nuts, before pelleting. Total amount used is such as to administer to an average animal $2 \times 10^{10}$ pfu/day.

Formulations are prepared for distribution into forage and prepared feedstuffs, for example, 500 grams per tonne of forage for dry powders, or suitably dissolved in drinking water to provide the required dosage.

EXAMPLE 2

Effect of Phage on Weight Gain in Cattle

Two groups of steers (Breed: Limousin x Friesian) were used in a weight-gain trial, and divided into two groups comprising 7 control and 7 treatment animals.

Each animal was fed daily:
4 kg Barley;
4 kg Beef Grower Nuts;
(14% Protein, 4% Oil, 9% Fibre, 9% Ash);
80 gm Mineral Supplement; and
Ad lib barley straw

In addition, the Test group was also fed a mixture of bacteriophages specific for Streptococcus bovis - (now renamed Streptococcus equinus) and Streptococcus durans (now renamed Enterococcus durans) to give a daily individual dose of $2 \times 10^{10}$ pfu (plaque-forming units).

The bacteriophage formulation used was prepared according to Example 1 a). A daily sachet of 120 gms was mixed with the total feed needed for the 7 animals in the treatment group.

The trial proceeded for 8 weeks, keeping a record of each animal's total feed intake. At the beginning of the trial, each animal was weighed and allocated randomly to one of the groups. At 8 weeks, each animal was re-weighed.

The results are shown in the following Table.

TABLE

| After 8 Weeks | Control Group | Test Group |
|---|---|---|
| Mean Liveweight gain (kg) | 72.710 | 92.860 |
| Total dry matter intake (kg) | 856.800 | 856.800 |
| Feed conversion | 0.247 | 0.304 |
| Daily liveweight gain (kg) | 1.298 | 1.658 |

The liveweight gain of the Test group was statistically highly significant (p = <o. 01), clearly demonstrating the advantage of the preparations of the present invention, even for the treatment of healthy livestock.

**Claims**

1. A method for the cosmetic treatment of a ruminant, especially a cow, comprising administering at least one variety of bacteriophage specific for a lactic acid bacterium, especially S. equinus or E. durans, to said ruminant in a manner, such as orally, adapted to introduce the bacteriophage into the digestive tract, particularly the rumen, of the ruminant.

2. A foodstuff, such as beef-grower nuts or water, or an ingredient thereof, for a ruminant, especially a cow, characterised in that the foodstuff contains at least one variety of bacteriophage specific for a lactic acid bacterium, especially S. equinus or E. durans.

3. A bacteriophage specific for a lactic acid bacterium, especially S. equinus or E. durans, for use in ruminant, especially cattle, therapy, particularly for the treatment of low rumen pH associated conditions, especially the treatment or prophylaxis of acidosis.

4. Use of at least one variety of bacteriophage specific for a lactic acid bacterium, especially S. equinus or E. durans, in the manufacture of a medicament for the treatment or prophylaxis of low rumen pH associated conditions, especially acidosis, in ruminants, especially cattle.

5. A preparation of a bacteriophage as defined in any preceding claim, wherein the preparation is a foodstuff containing about $10^2$ to about $10^{12}$ pfu/gm of bacteriophage.

6. A preparation of a bacteriophage for use in accordance with any preceding claim, wherein the preparation is adapted for the administration of between about $10^8$ and about $10^{12}$, especially about $10^{10}$, pfu/animal/day of bacteriophage.

7. A preparation according to claim 5 and/or 6 comprising at least two varieties of bacteriophage.

8. A method according to claim 1, wherein the variety of bacteriophage administered is varied over the treatment period.

9. A composition of preparations according to claims 5, 6 and/or 7, wherein there are at least two preparations which contain a differing selection of bacteriophage.

10. A bacteriophage for use in ruminant therapy.